# EUROPÄISCHE PATENTSCHRIFT

(11) **EP 3 261 485 B1**
(45) Veröffentlichungstag und Bekanntmachung des Hinweises auf die Patenterteilung: **28.08.2019**
(21) Anmeldenummer: 16703537.7
(22) Anmeldetag: 08.02.2016
(51) Int. Cl.: A45D 40/00, A61Q 19/00, A61K 8/34, A61K 8/36, A61K 8/37, A61K 8/39, A61K 8/891, B65D 43/02

(54) **KOSMETISCHES PRODUKT UMFASSEND EINE METALLDOSE UND DEREN INHALT**
COSMETIC PRODUCT HAVING A METAL CAN, AND THE CONTENTS OF SAME
PRODUIT COSMÉTIQUE COMPRENANT UNE BOÎTE MÉTALLIQUE ET SON CONTENU

(30) Priorität: 27.02.2015 DE 102015203565
(43) Veröffentlichungstag der Anmeldung: 03.01.2018
(73) Patentinhaber: Beiersdorf AG, 20253 Hamburg (DE)
(72) Erfinder: BAUER, Anja, 22459 Hamburg (DE); KALLMAYER, Volker, 22393 Hamburg (DE); STEIDLE, Peter, 22043 Hamburg (DE); STANGE, Klaus-Peter, 21465 Wentorf (DE)
(86) Internationale Anmeldenummer: PCT/EP2016/052612
(87) Internationale Veröffentlichungsnummer: WO 2016/134962

(56) Entgegenhaltungen:
- WO-A1-2008/125238
- DE-U- 7 102 103
- FR-A5- 2 185 335

## Beschreibung

Die Erfindung betrifft ein kosmetisches Produkt umfassend eine Metalldose aus einem Behälterteil mit Drehgewinde (Behältergewinde), in dessen Innerem eine kosmetische Zubereitung aufbewahrt wird, sowie einem Deckelteil aus Metall mit einem Gewinde, (Deckelgewinde), wobei der Deckelteil vermittels des Deckelgewindes und des Behältergewindes durch Drehbewegung auf den Behälterteil verschließend auf- und zuschraubbar, also durch Schraubverschluss öffnen- und wieder verschließbar ausgestaltet ist.

Kosmetische Produkte umfassend Metalldosen mit Inhalt, beispielsweise Cremes, Fette und dergleichen werden seit mindestens hundert Jahren verwendet, siehe beispielsweise WO 2008/125238 A1.

Nachteilig bei diesen bekannten Blechdosen ist es, dass entweder die Wahl des Wandungsmaterials eingeschränkt ist, da durch Schraubbewegung des Deckels auf dem Behälterteil nahezu zwangsläufig ein Abrieb entsteht, der zu unschönen Schmutzerscheinungen führt, oder dass Behälter ohne Schraubgewinde gewählt werden müssen, was aber zu Dichtigkeitsproblemen führen kann.

Aufgabe der Erfindung war es, eine Metalldose mit Profilrand zu schaffen, welcher eine kreisrunde Planfläche aufweist und insbesondere zum Versiegeln geeignet ist und dabei die geschilderten Nachteile des Standes der Technik nicht aufweist.

Gelöst werden diese Aufgaben erfindungsgemäß durch ein kosmetisches Produkt umfassend
eine Metalldose, umfassend
aa) einen metallenen Behälterteil (A) mit Drehgewinde (A'),
   aaa) in dessen Innerem sich eine kosmetische Zubereitung (Z) befindet, sowie
bb) einen Deckelteil (D) mit einem Gewinde, (D') aus demselben Metall wie der Behälterteil,
   wobei der Deckelteil (D) vermittels des Deckelgewindes (D') und des Behältergewindes (A') durch Drehbewegung auf den Behälterteil (A) verschließend auf- bzw. abschraubbar, also durch Schraubbewegung öffnen- und wieder verschließbar ausgestaltet ist,
   dadurch gekennzeichnet, dass die kosmetische Zubereitung eine Emulsion aus
cc) einer oder mehreren hydrophilen Phasen
dd) einer oder mehreren lipophilen Phasen
   umfasst, wobei
ee) bei welchen die Lipidphase mindestens ein Lipid mit einer Viskosität von weniger als 15 mPa·s (bei 25 °C), welches einen Spreitwert von mindestens 700 mm²/10 Minuten (bei 25 °C) aufweist, enthält.

Spreiten ist die oft erwünschte, in anderen Situationen auch oftmals unerwünschte vorwiegend auf Kapillarkräften beruhende Eigenschaft niedrigviskoser Öle, sich besonders leicht auf Unterlagen oder auch auf der Haut dünnschichtig zu verteilen. Dies kann bei der Hautpflege von Vorteil sein. Nachteilig kann sich diese Eigenschaft bei der Verpackung derartiger Öle oder diese enthaltender Zubereitungen auswirken. Ein Maß für das Spreitvermögen ist der Spreitungskoeffizient, der beispielsweise bei Entschäumern und Schaumverhütungsmitteln besonders hohe Werte annimmt.

Barry und Grace entwickelten eine Methode zur Bestimmung des Spreitverhaltens (J. Pharmac. Sci. 61, 335 [1972] und Beyer entwickelte ein Modell-Testsystem zur Prüfung des Spreitverhaltens (Arch. Pharm. [Weinh.] 310, 729 [1977]; Chem.Abstr. 88, Nr. 12-79017 [1978]). Über das Spreiten von Salben am Modell berichtet Beyer ferner in Arch. Pharm. 310, 473 und 858 (1977); Zbl. Pharm. 118, 51 (1979). Über das Spreitvermögen verschiedener flüssiger Hilfsstoffe auf Basis von Fetten bzw. fettähnlichen Stoffen berichten Pascale et.al. (Cosmet. Toiletries 100, Nr. 10, 75 [1985]).

Die Maßeinheit des Spreitungskoeffizienten ist die des Quotienten aus der Spreitungsfläche, über die die Spreitung erfolgt, und der Spreitungszeit, in der die Spreitung erfolgt. Sie wird üblicherweise in [mm²/ 10 Minuten] angegeben.

Im Rahmen der vorliegenden Offenbarung wird als Oberbegriff für Fette, Öle, Wachse und dergleichen gelegentlich der Ausdruck "Lipide" verwendet, wie dem Fachmanne durchaus geläufig ist. Auch werden die Begriffe "Ölphase" und "Lipidphase" synonym angewandt.

Bevorzugt enthalten erfindungsgemäße Zubereitungen bis zu 35 Gew.-% einer Lipidphase.

Vorteilhaft werden die erfindungsgemäß verwendeten Öle gewählt aus der Gruppe der Substanzen, die in der folgenden Tabelle aufgelistet sind:

**Tabelle 1**

| Handelsname | Name analog INCI | Viskosität mPas | Spreitfähigkeit (20/µl/Rotbandfilter) |
|---|---|---|---|
| | | mPa·s | mm²/10 Min. |
| Solvent IH | Isohexadecan | 8 | 990 |
| | Isoeikosan | 12 | 800 |
| Cegesoft® C24 | Octylpalmitate | 11 | 910 |
| | Isopropylstearat | 9 | 910 |
| Estol® 1540 EHC | Octylcocoat | 10 | 930 |
| Finsolv® TN | C₁₂₋₁₅ Alkylbenzoate | 14 | 730 |
| Cetiol® OE | Dicaprylylether | 8 | 1.020 |
| DUB DNPG | Neopentyl-Glycol-diheptanoat | 13 | 830 |
| Miglyol® 840 | Propylenglycol-dicaprylat/dicaprat | 12 | 855 |
| DC Fluid 345 | Cyclomethicon | 5 | 770 |
| | **Isopropylpalmitat** | 7,1 | 1.590 |
| Cetiol® B | Dibutyladipat | 5,5 | 935 |
| DUB VCI 10 | Isodecylneopentanoat | 3,9 | 962 |
| Cetiol® CC | Dicaprylylcarbonat | 7,26 | 875 |
| ***Cetiol***® ***S*** | Dioctylcyclohexan | 14,41 | 723 |
| | Dihexylcarbonat | 3,8 | 1.056 |
| | Dihexylether | 1,87 | 1.174 |
| Ecolane® 130 | Cycloparaffin | 5,22 | 908 |
| Softcutol® O | Ethoxydiglycololeate | 13,07 | 804 |
| Transcutol® CG | Ethoxydiglycol | 4,14 | 999 |
| Dermofeel® BGC | Butylenglycol-caprylat/caprat | 12 | 800 |
| Prisorine® 2036 | Octylisostearat | 15 | 800 |
| Tegosoft® SH | Stearylheptanoat | 13 | 755 |
| Tegosoft® DC | Decylcocoat | 10,3 | 788 |
| Transcutol® P | Ethoxydiglycol | 4,27 | 962 |
| Arlasolv® DMI | Dimethylisosorbid | 10 | 880 |

Eine solche Metalldose weist eine ausreichend breite, ebene Fläche an der Oberkante zum Versiegeln auf, so dass in an sich bekannter Weise Aufleger fest auf die Dose gesiegelt werden können. Dazu wird auf die gefüllte Dose beispielsweise eine flexible Folie aus Metall, Kunststoff oder ihrem Verbund mittels Wärmekontakt-Verschweißung oder Ultraschall aufgesiegelt, wobei der vor der Formung der Dose applizierte Innenschutzlack thermoplastisch siegelfähige oder ultraschallschweißfähige Eigenschaften hat.

Es kann auch nachträglich eine geeignete Versiegelungsmasse, wie hochschmelzendes Wachs, Klebstoff, Lack oder dergleichen auf die kreisringförmige Planfläche des Profilrandes der fertig geformten Dose aufgetragen werden, worauf die Folie aufgesiegelt wird, beispielsweise durch Druck, Wärme, Wärmestrahlung, Ultraschall oder dergleichen, wobei auch eine Kombination dieser Maßnahmen angewendet werden kann, wie dies zum Stand der Technik gehört. Die versiegelte Dose kann sodann mit einem Deckel in üblicher Weise verschlossen werden.

Vorteilhaft ist auf der Innenseite der Dose in an sich bekannter Weise ein Innenschutz angebracht, insbesondere aus einem siegelfähigen Lack oder dergleichen, wobei dieser Innenschutz vorteilhaft bereits vor der Formung der Dose und des Rollrandes angebracht ist Gemäß einer bevorzugten Ausführungsform läuft seitlich um die Dose eine Sicke, die zu ihrer Versteifung und ggf. als Anschlag für einen aufzusetzenden Deckel dient, wie dies an sich bekannt ist. Weitere oder andere Sicken können in an sich bekannter Weise vorgesehen sein. Zudem ist vorteilhaft der Boden der erfindungsgemäßen Dose leicht nach innen gewölbt, kann aber auch, wenn erwünscht, eben sein.

Als Material wird für die erfindungsgemäße Dose vorteilhaft Aluminium oder eine AluminiumLegierung verwendet, wobei aber auch Stahlblech oberflächenbehandelt wie verzinnt, verchromt, aluminiert, lackiert, kunststoffbeschichtet etc., geeignet ist.

Die Herstellung der erfindungsgemäßen Dosen kann in an sich bekannter Weise erfolgen. Insbesondere wird der flanschförmige Rand des auf einem umlaufenden Futter festgespannten, tiefgezogenen Dosenkörpers durch ein tangential zu seiner Mittelachse wirkendes, als Profilscheibe ausgebildetes Drückwerkzeug mit seiner Schnittkante nach außen, rotierend zu einem Profilrand gedrückt, der danach an seiner Oberseite ein halbrundes Profil aufweist, welches nachfolgend mit einem Planflächenglattwalzwerkzeug unter axialem Vorschub plastisch verformt und dabei flachgedrückt wird, so dass eine vollkommen ebene, kreisringförmige Planfläche entsteht Eine vorteilhafte Ausführungsform der erfindungsgemäßen Dose sowie eine Dose des Standes der Technik sind in der Zeichnung dargestellt.

Die erfindungsgemäßen kosmetischen oder dermatologischen Zubereitungen können wie üblich zusammengesetzt sein und zur Behandlung, der Pflege und der Reinigung der Haut und/oder der Haare und als Schminkprodukt in der dekorativen Kosmetik dienen. Sie enthalten bevorzugt 0,001 Gew.-% bis 10 Gew.-%, bevorzugt 0,05 Gew.-% bis 5 Gew.-%, insbesondere 0,1 bis 2,0 Gew.-%, bezogen auf das Gesamtgewicht der Zubereitungen, an erfindungsgemäß verwendeten Wirkstoffkombinationen.

Es ist erfindungsgemäß bevorzugt, den erfindungsgemäß verwendeten Wirkstoffkombinationen bzw. kosmetischen oder dermatologischen Zubereitungen, solche Wirkstoffkombinationen enthaltend Komplexbildner zuzufügen.

Komplexbildner sind an sich bekannte Hilfsstoffe der Kosmetologie bzw. der medizinischen Galenik. Durch die Komplexierung von störenden Metallen wie Mn, Fe, Cu und anderer können beispielsweise unerwünschte chemische Reaktionen in kosmetischen oder dermatologischen Zubereitungen verhindert werden.

Komplexbildner, insbesondere Chelatoren, bilden mit Metallatomen Komplexe, welche bei Vorliegen eines oder mehrerer mehrbasiger Komplexbildner, also Chelatoren, Metallacyclen darstellen. Chelate stellen Verbindungen dar, in denen ein einzelner Ligand mehr als eine Koordinationsstelle an einem Zentralatom besetzt. In diesem Falle werden also normalerweise gestreckte Verbindungen durch Komplexbildung über ein Metall-Atom od. -Ion zu Ringen geschlossen. Die Zahl der gebundenen Liganden hängt von der Koordinationszahl des zentralen Metalls ab. Voraussetzung für die Chelatbildung ist, dass die mit dem Metall reagierende Verbindung zwei oder mehr Atomgruppierungen enthält, die als Elektronendonatoren wirken.

Der oder die Komplexbildner können vorteilhaft aus der Gruppe der üblichen Verbindungen gewählt werden, wobei bevorzugt mindestens eine Substanz aus der Gruppe bestehend aus Weinsäure und deren Anionen, Citronensäure und deren Anionen, Aminopolycarbonsäuren und deren Anionen (wie beispielsweise Ethylendiamintetraessigsäure (EDTA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen).

Der oder die Komplexbildner sind erfindungsgemäß vorteilhaft in kosmetischen oder dermatologischen Zubereitungen bevorzugt zu 0,01 Gew.-% bis 10 Gew.-%, bevorzugt zu 0,05 Gew.-% bis 5 Gew.-%, insbesondere bevorzugt zu 0,1 bis 2,0 Gew.-%, bezogen auf das Gesamtgewicht der Zubereitungen, enthalten.

Zur Anwendung werden die kosmetischen und dermatologischen Zubereitungen erfindungsgemäß in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-ÖI (W/O) oder vom Typ Öl-in-Wasser (O/W), eine multiple Emulsionen, beispielsweise vom Typ Wasser-in-ÖI-in-Wasser (W/O/W), ein Gel, einen festen Stift, eine Salbe oder auch ein Aerosol darstellen. Es ist auch vorteilhaft, Isoquercitrin in verkapselter Form darzureichen, z.B. in Kollagenmatrices und anderen üblichen Verkapselungsmaterialien, z.B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt.

Es ist auch möglich und vorteilhaft im Sinne der vorliegenden Erfindung, erfindungsgemäß verwendete Wirkstoffkombinationen in wässrige Systeme bzw. Tensidzubereitungen zur Reinigung der Haut und der Haare einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feucht haltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Die Lipidphase kann vorteilhaft gewählt werden aus folgender Substanzgruppe:
- Mineralöle, Mineralwachse
- Öle, wie Triglyceride der Caprin- oder der Caprylsäure, Esteröle, wie Dicaprylylether, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren oder natürliche Wachse, wie Shea Butter;
- Alkylbenzoate;
- Silikonöle wie Dimethicone, Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Dimethicon als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylhexlglycerin, der Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, TR2, TR1 jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wässrig-alkoholischen oder alkoholischen Gelen vorzugweise ein Polyacrylat ist.

Vorteilhaft können erfindungsgemäße Zubereitungen außerdem weitere Substanzen enthalten, die UV-Strahlung im UVA- und/oder UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Die folgenden Beispiele sollen die Erfindung näher beschreiben. Die Angaben beziehen sich, sofern nichts anderes angegeben ist, auf Gew.-%, bezogen auf das Gesamtgewicht der Zusammensetzung (Z).

| **Beispiel Nr.** | **1** | **2** | **3** | **4** | **5** |
|---|---|---|---|---|---|
| | | | | | |
| Palmitinsäure + Stearinsäure + Myristinsäure + Arachidionsäure + Ölsäure | 0,9 | | | 1 | |
| Polyglyceryl-10 Stearat | | 1,2 | 0,8 | | 1,2 |
| Glycerylstearat | 0,85 | 1,2 | 0,8 | 0,99 | 1,2 |
| Cetylstearylalkohol | | 3,2 | 2,4 | | 3,2 |
| Cetylalkohol | 1 | | | 1,2 | |
| Dimethicone | 0,25 | 0,9 | 0,9 | 0,25 | 0,9 |
| C12-15 Alkyl Benzoate | | 2,5 | 1,8 | | 2,2 |
| Isopropylstearat | 2,9 | | | 3,1 | |
| Isopropylpalmitat | | 3,9 | 3 | | 3,9 |
| Carbomer | 0,7 | 0,2 | 0,2 | 0,7 | 0,2 |
| Acrylates/C10-30 Alkyl Acrylate Crosspolymer | 0,04 | 0,4 | 0,2 | 0,06 | 0,1 |
| Xanthan Gum | | 0,2 | | | |
| Glycerin | 10 | 10 | 5 | 5 | 10 |
| Phenoxyethanol | 0,25 | 0,5 | 0,5 | 0,25 | 0,5 |
| Alcohol Denat. 96 % | 10 | 3 | 3 | 10 | 3 |
| Ethylhexylglycerin | | 0,5 | 0,5 | | 0,5 |
| Distärkephosphat | 0,5 | 0 | 1 | 1,5 | 0 |
| Wasser + Trinatrium EDTA | | 1 | 1 | | 1 |
| Octocrylen | | 2 | 1 | | 2,5 |
| Butylmethoxydibenzoylmethan | | 2 | 1 | | 3 |
| Phenylbenzimidazolsulfonsäure | | 2,25 | | | |
| Wasser + NaOH (45%) | 0,65 | 1,15 | 0,3 | 0,85 | 0,35 |
| Wasser | ad 100 | ad 100 | ad 100 | ad 100 | ad 100 |

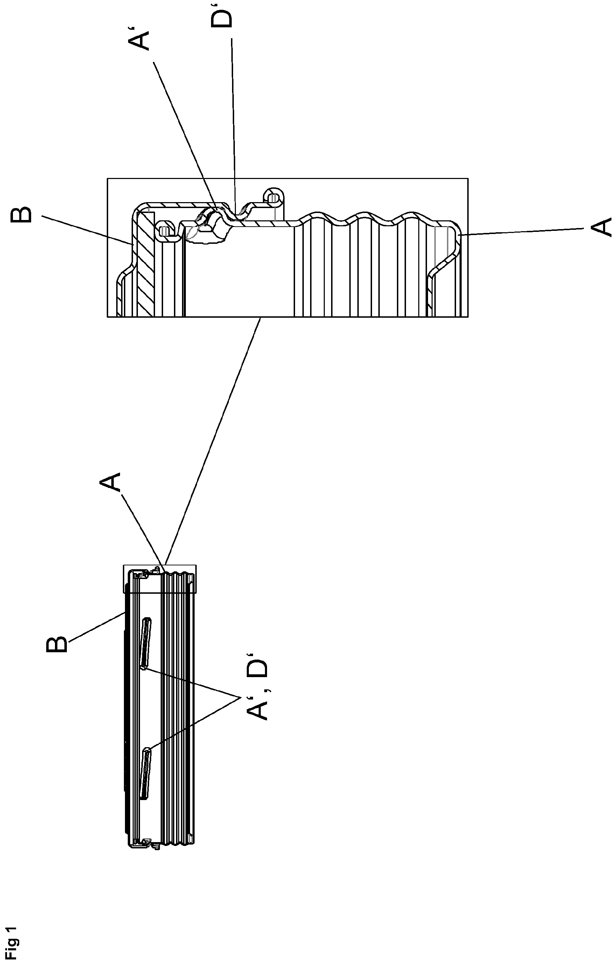

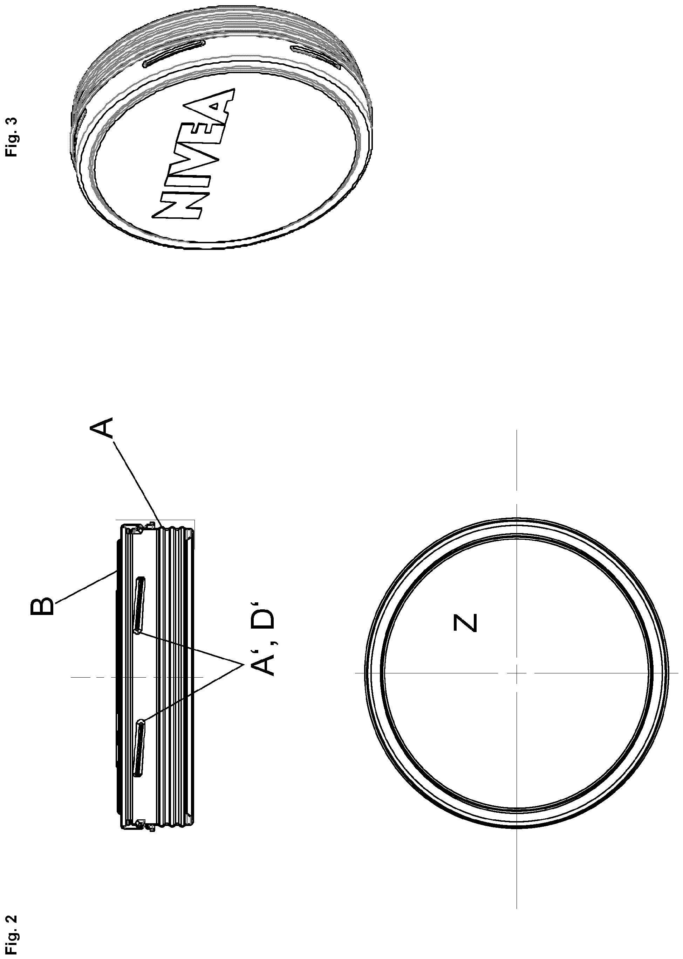

Die Zubereitungen gemäß den Beispielen werden in Aluminiumdosen gemäß den Figuren 1 - 3 abgefüllt.
Fig. 1 zeigt eine Schnittzeichnung einer erfindungsgemäß verwendeten Metalldose, umfassend
   aa) einen metallenen Behälterteil (A) mit Drehgewinde (A'),
      aaa) in dessen Innerem sich eine kosmetische Zubereitung (Z) befindet, sowie
   bb) einen Deckelteil (D) mit einem Gewinde, (D') aus demselben Metall wie der Behälterteil,
   wobei der Deckelteil (D) vermittels des Deckelgewindes (D') und des Behältergewindes (A') durch Drehbewegung auf den Behälterteil (A) verschließend auf- bzw. abschraubbar, also durch Schraubbewegung öffnen und wieder verschließbar ausgestaltet ist.
   Ferner wird in Fig. 1 ein Detail der Wandung der Dose bzw. des Deckels, wo insbesondere Gewindeteile A' und D' herausgehoben sind.
Fig. 2 zeigt die Schnittzeichnung aus Fig. 1 wiederholt und einen waagereichten Schnitt durch das komplette kosmetische Produkt, also die Dose mit der kosmetischen Zubereitung (Z)
Fig. 3 zeigt eine perspektivische Zeichnung des kosmetischen Produktes mit einem Werbeschriftzug.

## Patentansprüche

1. Kosmetisches Produkt umfassend
eine Metalldose, umfassend
aa) einen metallenen Behälterteil (A) mit Drehgewinde (A'),
aaa) in dessen Innerem sich eine kosmetische Zubereitung (Z) befindet, sowie
bb) einen Deckelteil (D) mit einem Gewinde, (D') aus demselben Metall wie der Behälterteil,
wobei der Deckelteil (D) vermittels des Deckelgewindes (D') und des Behältergewindes (A') durch Drehbewegung auf den Behälterteil (A) verschließend auf- bzw. abschraubbar, also durch Schraubbewegung öffnen- und wieder verschließbar ausgestaltet ist,
wobei die kosmetische Zubereitung eine Emulsion aus
cc) einer oder mehreren hydrophilen Phasen
dd) einer oder mehreren lipophilen Phasen
umfasst, wobei
ee) bei welchen die Lipidphase mindestens ein Lipid mit einer Viskosität von weniger als 15 mPa·s (bei 25 °C), welches einen Spreitwert von mindestens 700 mm²/10 Minuten (bei 25 °C) aufweist, enthält.

2. Kosmetisches Produkt nach Anspruch 1, **dadurch gekennzeichnet, dass** das oder die verwendeten Lipide gewählt wird oder werden aus der Gruppe folgender Substanzen:
Palmitinsäure, Stearinsäure, Myristinsäure, Arachidinsäure, Ölsäure, Glycerylstearat, Cetylalkohol, Cetearyl Alcohol, C12-15 Alkyl Benzoate, Polyglyceryl-10 Stearate, Isopropyl Stearate, Isopropyl Palmitate, Dimethicone.

3. Kosmetisches Produkt nach Anspruch 1 **dadurch gekennzeichnet, dass** die Metalldose aus Aluminium besteht, wobei auf der Innenseite der Dose in an sich bekannter Weise ein Innenschutz angebracht, insbesondere aus einem siegelfähigen Lack oder dergleichen, wobei dieser Innenschutz vorteilhaft bereits vor der Formung der Dose und des Rollrandes angebracht ist.

## Claims

1. Cosmetic product comprising
a metal can, comprising
aa) a metallic container part (A) with screw thread (A'),
aaa) within which there is a cosmetic preparation (Z), and
bb) a lid part (D) with a thread (D') made of the same metal as the container part, wherein the lid part (D) can be sealed or unscrewed by means of the lid thread (D') and the container thread (A') by rotating the container part (A), i.e. it is configured such that it may be opened and closed again by a screwing movement, wherein the cosmetic preparation comprises an emulsion composed of
cc) one or more hydrophilic phases
dd) one or more lipophilic phases,
wherein
ee) in which the lipid phase comprises at least one lipid having a viscosity of less than 15 mPa·s (at 25°C), which has a spreadability of at least 700 mm²/10 minutes.

2. Cosmetic product according to Claim 1, **characterized in that** the lipid(s) used is or are selected from the group of following substances: palmitic acid, stearic acid, myristic acid, arachidic acid, oleic acid, glyceryl stearate, cetyl alcohol, cetearyl alcohol, C12-15 alkyl benzoate, polyglyceryl-10 stearate, isopropyl stearate, isopropyl palmitate, dimethicone.

3. Cosmetic product according to Claim 1, **characterized in that** the metal can consists of aluminium, wherein internal protection is applied to the internal side of the can in a manner known per se, especially composed of a sealable coating material or the like, wherein this internal protection is advantageously already applied prior to the shaping of the can and of the rolled edge.

## Revendications

1. Produit cosmétique comprenant
une boîte métallique, comprenant
aa) une partie de récipient en métal (A) pourvue d'un taraudage (A'),
aaa) à l'intérieur de laquelle se trouve une préparation cosmétique (Z), ainsi que
bb) une partie de couvercle (D) pourvue d'un filetage, (D') étant composé du même métal que la partie de récipient,
la partie de couvercle (D) étant conçue pour être vissable sur la partie de récipient en la fermant au moyen du filetage de couvercle (D') et du taraudage du récipient (A') par un mouvement de rotation et dévissable de celle-ci, c'est-à-dire étant conçue de manière ouvrable et refermable par un mouvement de vissage, la préparation cosmétique comprenant une émulsion
cc) d'une ou de plusieurs phase(s) hydrophile(s)
dd) d'une ou de plusieurs phase(s) lipophile(s),
ee) dans lesquelles la phase lipidique contient au moins un lipide présentant une viscosité inférieure à 15 mPa.s (à 25°C), qui présente une valeur d'étalement d'au moins 700 mm²/10 minutes (à 25°C).

2. Produit cosmétique selon la revendication 1, **caractérisé en ce que** le ou les lipide(s) utilisé(s) est/sont choisi(s) dans le groupe des substances suivantes : acide palmitique, acide stéarique, acide myristique, acide arachidique, acide oléique, stéarate de glycéryle, alcool cétylique, alcool cétéarylique, benzoate de C₁₂₋₁₅-alkyle, stéarate de polyglycéryle-10, stéarate d'isopropyle, palmitate d'isopropyle, diméthicone.

3. Produit cosmétique selon la revendication 1, **caractérisé en ce que** la boîte métallique est constituée d'aluminium, une protection interne, en particulier composée d'une laque scellable ou similaire, étant disposée sur la face interne de la boîte d'une manière connue en soi, cette protection interne étant avantageusement déjà disposée avant la mise en forme de la boîte et du bord roulé.
